# FASCICULE DE BREVET EUROPEEN

(11) **EP 3 151 765 B1**
(45) Date de publication et mention de la délivrance du brevet: **21.11.2018**
(21) Numéro de dépôt: 15736538.8
(22) Date de dépôt: 03.06.2015
(51) Int. Cl.: A61B 17/43, A61D 19/02

(54) **DISPOSITIF PERFECTIONNE DE TRANSFERT ATRAUMATIQUE DE MATERIEL OU SUBSTANCE A BUT REPRODUCTIF, THERAPEUTIQUE OU DIAGNOSTIQUE DANS DES MAMMIFERES FEMELLES**
VERBESSERTE VORRICHTUNG FÜR DIE ATRAUMATISCHE ÜBERTRAGUNG EINES MATERIALS ODER STOFFES MIT REPRODUKTIVEM, THERAPEUTISCHEM ODER DIAGNOSTISCHEM ZWECK AUF WEIBLICHE SÄUGETIEREN
IMPROVED DEVICE FOR THE ATRAUMATIC TRANSFER OF A MATERIAL OR SUBSTANCE WITH A REPRODUCTIVE, THERAPEUTIC OR DIAGNOSTIC PURPOSE INTO FEMALE MAMMALS

(30) Priorité: 04.06.2014 FR 1455079
(43) Date de publication de la demande: 12.04.2017
(73) Titulaire: Elexinn, 89400 Migennes (FR)
(72) Inventeur: DECHERF, Agathe, 89400 Migennes (FR); DREVILLON, Pierrick, 89400 Migennes (FR)
(74) Mandataire: Cabinet Chaillot
(86) Numéro de dépôt international: PCT/FR2015/051474
(87) Numéro de publication internationale: WO 2015/185863

(56) Documents cités:
- WO-A1-03/065925
- FR-A- 1 472 139
- FR-A1- 2 705 880
- GB-A- 867 274
- US-A- 3 507 281

## Description

La présente invention concerne le domaine général du transfert, in vivo, de matériel ou substance à but reproductif, thérapeutique ou diagnostique dans une corne utérine d'un mammifère femelle, et notamment un bovin, et porte en particulier sur un dispositif pour un tel transfert, de préférence dans la partie supérieure d'une corne utérine.

On désigne, par l'expression « matériel ou substance à but reproductif » (ou de reproduction), de la semence ou un embryon, le dispositif de transfert en question étant alors un dispositif d'insémination artificielle ou de transfert d'embryon. Le matériel de reproduction pourra être contenu dans une paillette, comme cela est bien connu.

On désigne, par l'expression « matériel ou substance à but thérapeutique », tout agent visant au passage du sujet d'un état pathologique à un état normal, ou à la prévention d'un état pathologique. On peut ainsi mentionner, comme exemple, des antibiotiques intra-utérins profonds, des solutions antiseptiques ou des solutions de lavage isotoniques.

On désigne, par l'expression « matériel ou substance à but diagnostique », tout agent permettant ou aidant un praticien à établir un diagnostic. On peut ainsi mentionner, comme exemple, des substances de marquage, telles que le bleu de méthylène.

Concernant le transfert de matériel de reproduction, on connaît des dispositifs d'insémination artificielle ou de transfert d'embryon ayant un ensemble de gaines destinées à coulisser les unes par rapport aux autres, la gaine la plus interne comprenant le matériel de reproduction à déposer, et un piston permettant de pousser ledit matériel de reproduction hors de la gaine.

Un tel dispositif est divulgué dans le brevet FR 2705880. Il comprend un embout flexible, fixé à l'extrémité proximale d'un tube allongé, avec lequel il forme une gaine interne, l'embout ayant un passage communiquant avec une paillette engagée dans le tube allongé. La partie avant de l'embout est de forme conique afin de le faire passer dans le canal cervical d'un sujet femelle sans endommager celui-ci, l'embout flexible étant ensuite avancé dans une corne utérine jusqu'à atteindre sa partie supérieure. Le dispositif comprend également des moyens de protection contre une contamination extérieure, constitués d'un premier moyen enfermant le tube allongé et d'un second moyen entourant l'embout et le premier moyen. Un piston est disposé dans le tube allongé et une pression sur celui-ci permet d'envoyer le contenu de la paillette dans l'embout flexible, lorsque le dispositif est inséré dans le canal vaginal du sujet.

Cependant, le dispositif selon le brevet FR 2705880 ne permet pas d'expulser la totalité du contenu de la paillette. En effet, le diamètre du piston est plus important que le diamètre interne de l'embout flexible. Le piston ne pénètre donc pas dans ce dernier, et le volume interne de l'embout flexible, qui est suffisamment long pour atteindre la partie supérieure de la corne utérine, constitue ainsi un important volume mort. Ce problème se poserait également dans le cas, non envisagé dans le brevet FR 2705880, du transfert de matériel ou substance à but thérapeutique ou diagnostique.

En outre, il est nécessaire, lors de l'utilisation de tout dispositif de transfert de matériel ou substance à but reproductif, thérapeutique ou diagnostique, de ne pas blesser l'animal. En effet, par exemple dans le cas de l'insémination artificielle, le traumatisme associé à une blessure engendrera une réaction inflammatoire locale qui sera spermicide, ce qui limitera les chances de succès de fécondation. Il convient donc d'éviter que le dispositif soit susceptible, par ses dimensions et/ou son fonctionnement, de blesser l'animal. En d'autres termes, le dispositif doit être atraumatique.

La présente invention vise à surmonter les inconvénients mentionnés ci-dessus et à proposer un dispositif de transfert atraumatique de matériel ou substance à but reproductif, thérapeutique ou diagnostique permettant de déposer la totalité dudit matériel ou substance dans la région adéquate, pour une meilleure efficacité du matériel ou substance, comme par exemple un meilleur taux de succès de l'insémination artificielle ou du transfert d'embryon, sans risquer de blesser l'animal.

La présente invention a pour objet un dispositif de transfert atraumatique de matériel ou substance à but reproductif, thérapeutique ou diagnostique dans un mammifère femelle, notamment un bovin, permettant un transfert dans une région, dite de transfert, d'une corne utérine d'un mammifère femelle, comprenant :
- un ensemble corps ;
- une gaine extérieure ayant une extrémité proximale ouverte et une extrémité distale ouverte, la gaine extérieure étant solidaire de l'ensemble corps et apte à être introduite dans le vagin du mammifère femelle et franchir le col de l'utérus du mammifère femelle, de préférence jusqu'à venir au début de l'une des deux cornes utérines ;
- une gaine intérieure ayant une extrémité proximale ouverte, une extrémité distale dans laquelle est ménagée au moins une ouverture dite de transfert, et un canal interne s'étendant entre les extrémités proximale et distale de la gaine intérieure et apte à recevoir un matériel, ou une substance, à but reproductif, thérapeutique ou diagnostique à transférer, la gaine intérieure étant reçue de manière coulissante dans la gaine extérieure et étant souple de façon à être apte à être étendue, à partir de l'extrémité distale de la gaine extérieure, dans la corne utérine du mammifère femelle jusqu'à ce que l'extrémité distale de la gaine intérieure se situe dans la région de transfert ; et
- un piston destiné, après mise en place du matériel ou substance à transférer dans la gaine intérieure, à être introduit dans la gaine intérieure de manière à pouvoir coulisser dans celle-ci et à conduire, par un mouvement de translation relatif entre le piston et la gaine intérieure, le matériel ou substance à transférer vers l'extrémité distale de la gaine intérieure, par laquelle le matériel ou substance à transférer sera déposé dans la région de transfert,
dispositif caractérisé par le fait qu'une partie, dite distale, du piston est souple et apte à s'étendre dans la partie, dite distale, de la gaine intérieure qui est destinée à s'étendre dans la corne utérine et par le fait que le dispositif comprend des moyens de déplacement conjoint de la gaine intérieure et du piston jusqu'à la région de transfert, avec ladite partie distale du piston se situant à l'intérieur de ladite partie distale de la gaine intérieure, et des moyens de rétractation de la gaine intérieure sur le piston au niveau de la région de transfert, de façon à faire sortir le matériel ou substance à transférer par l'extrémité distale de la gaine intérieure, sous la poussée du piston.

On entend par « déplacement conjoint » un déplacement simultané de la gaine intérieure et du piston, sans déplacement relatif entre la gaine intérieure et le piston. Ainsi, le déplacement conjoint de la gaine intérieure et du piston, qui consiste à étendre la partie distale de la gaine intérieure dans une corne utérine, ne provoque pas le dépôt du matériel ou substance à transférer.

Le mouvement de rétractation de la gaine intérieure sur la partie distale du piston permet de faire sortir le matériel ou substance à transférer tout en évitant une blessure de l'animal, puisque la sortie du matériel ou substance à transférer n'est plus obtenue par un mouvement de poussée du piston en direction de la partie supérieure de la corne utérine. Le transfert du matériel ou de la substance est donc atraumatique.

De plus, le problème du volume mort est résolu par le fait que la partie distale du piston s'étend dans la partie de la gaine intérieure qui s'étend dans la corne utérine.

Selon un mode de réalisation préféré de la présente invention, l'ensemble corps comprend une tige s'étendant dans la direction à l'opposé de la gaine extérieure et sur laquelle est monté coulissant un ensemble navette qui comprend une navette primaire, montée coulissante sur ladite tige et à laquelle le piston est fixé, et une navette secondaire, montée coulissante sur ladite navette primaire et à laquelle l'extrémité proximale de la gaine intérieure est fixée, la navette primaire et la navette secondaire constituant lesdits moyens de déplacement conjoint et lesdits moyens de rétractation.

La gaine intérieure peut ainsi être avancée dans la corne utérine sans mouvement relatif vis-à-vis du piston, par le déplacement de la navette primaire en direction de la gaine extérieure avec la navette secondaire immobile par rapport à la navette primaire, puis, après arrivée de l'extrémité distale de la gaine intérieure à la région de transfert, la gaine intérieure peut être rétractée sur le piston par coulissement de la navette secondaire à l'opposé de la gaine extérieure tandis que le piston reste immobile vis-à-vis de la gaine extérieure ou est avancé, conjointement avec la gaine intérieure, par coulissement de la navette primaire en direction de la gaine extérieure.

De préférence, la gaine intérieure peut comprendre une partie proximale, à l'intérieur de la gaine extérieure, et la partie distale souple, de préférence d'une longueur supérieure à 130 mm, de façon davantage préférée d'une longueur de 255,5 mm, destinée à s'étendre dans la corne utérine, la partie proximale étant moins souple que la partie distale.

Prévoir une telle longueur minimale permet à l'extrémité distale de la gaine intérieure d'aller suffisamment profondément dans une corne utérine pour un bon effet reproductif, thérapeutique ou diagnostique, et, dans le cas de la longueur préférée de 255,5 mm, d'atteindre la partie supérieure d'une corne utérine pour un meilleur effet. Ainsi, dans le cas où le dispositif est utilisé comme dispositif d'insémination artificielle, la semence peut être déposée à proximité du réservoir spermatique, pour un taux de succès amélioré.

Selon un mode de réalisation particulier du dispositif selon la présente invention, approprié au transfert de matériel ou substance à but reproductif, thérapeutique ou diagnostique contenu(e) dans l'espace intérieur, de section transversale donnée, d'une paillette, le canal interne de la gaine intérieure est apte à recevoir une telle paillette, le piston est apte à traverser la paillette pour en faire sortir le matériel ou substance à transférer et la gaine intérieure comprend des moyens d'arrêt en translation de la paillette dans la direction de l'extrémité distale, les moyens d'arrêt en translation de la paillette étant notamment formés par un épaulement intérieur dans la gaine intérieure, le cas échéant situé à la jonction entre les parties proximale et distale de la gaine intérieure, et, de préférence, centré sur l'axe de la gaine intérieure.

De préférence, lorsque le dispositif selon la présente invention comprend les moyens d'arrêt en translation formés par un épaulement intérieur et est pour une utilisation avec une paillette dont l'espace intérieur a une section transversale égale à la section de passage du canal interne au niveau de l'épaulement intérieur, sont avantageusement prévus des moyens de centrage de la paillette sur l'axe de la gaine intérieure lorsque la paillette est mise en butée contre les moyens d'arrêt en translation, lesdits moyens de centrage étant notamment formés par un rétrécissement de la section de passage du canal interne de la gaine intérieure au voisinage de l'épaulement, côté extrémité proximale de la gaine intérieure, le rétrécissement étant centré sur l'axe de la gaine intérieure.

L'ensemble corps peut comprendre un nez dans lequel sont ménagés un passage traversant, à partir d'une extrémité duquel s'étend la gaine extérieure, sur un premier côté du nez, et un trou débouchant sur un second coté du nez, opposé audit premier côté, et à partir duquel s'étend la tige de l'ensemble corps, l'axe du passage traversant et l'axe du trou étant parallèles et coplanaires.

La gaine extérieure peut être reliée au nez par un raccord, notamment de type Luer.

La navette secondaire peut comprendre un passage traversant pour le piston et la gaine intérieure peut être reliée à la navette secondaire par un raccord, notamment de type Luer.

De préférence, les extrémités distales de la gaine extérieure et de la gaine intérieure sont exemptes d'arêtes et bords tranchants, lesdites extrémités distales étant, de préférence, bombées, de façon à faciliter la progression des gaines extérieure et intérieure dans l'animal sans blesser ce dernier.

L'extrémité distale de la gaine intérieure peut être formée par un embout ayant deux sorties latérales permettant de déposer le matériel ou substance à transférer dans la région de transfert, en particulier de la semence en direction des cryptes spermatiques, ledit embout présentant, de préférence, une forme bombée à son extrémité.

L'extrémité distale de la gaine extérieure peut être formée par un embout en matière souple, en forme d'ogive et percé d'une ouverture en croix.

Les gaines intérieure et extérieure peuvent être en matière plastique.

Le dispositif selon la présente invention comprend avantageusement une chemise sanitaire, qui entoure la gaine extérieure et vient se fixer sur l'extrémité de celle-ci, ladite chemise sanitaire permettant de maintenir le dispositif propre jusqu'à son entrée dans le col utérin, ladite chemise sanitaire étant apte à être percée pour permettre l'extension de la gaine intérieure à partir de la gaine extérieure. La chemise sanitaire pourra être du type décrit dans la demande de brevet européen EP0093630.

Le piston peut être formé par une combinaison d'une tige, notamment métallique, et d'un câble, respectivement côté proximal et côté distal du dispositif, le câble formant la partie distale du piston destinée à s'étendre dans la partie de la gaine intérieure destinée à s'étendre dans la corne utérine du mammifère.

En variante, le piston peut être formé par un jonc rigide, formant la partie proximale du piston par laquelle il est, le cas échéant, fixé à la navette primaire, par une tige souple et apte à traverser une paillette, la tige, qui est notamment métallique, s'étendant à partir d'une extrémité distale du jonc, et par un câble s'étendant à partir d'une extrémité distale de la tige, le câble et la région d'extrémité distale de la tige formant la partie distale du piston destinée à s'étendre dans la partie de la gaine intérieure destinée à s'étendre dans la corne utérine du mammifère.

La longueur du piston peut être sensiblement égale à celle de la gaine intérieure.

Selon un mode de réalisation particulier, la navette secondaire est munie d'un premier aimant, la navette primaire est munie, dans sa région d'extrémité distale, d'un deuxième aimant, et la tige est munie d'un troisième aimant dans sa région d'extrémité proximale, les aimants étant configurés pour être aptes à être alignés les uns avec les autres dans une position donnée des navettes primaire et secondaire et s'attirer les unes les autres de façon à maintenir en position les navettes primaire et secondaire par rapport à la tige.

De préférence, la tige porte à son extrémité distale une butée, de préférence une butée reçue dans un trou traversant de la navette primaire, par lequel la navette primaire est montée coulissante sur la tige, le trou traversant comprenant, de préférence à son extrémité distale, un épaulement présentant une surface d'arrêt pour ladite butée.

Est également divulgué par la présente un procédé pour transférer du matériel ou substance à but reproductif, thérapeutique ou diagnostique dans une région, dite de transfert, d'une corne utérine d'un mammifère femelle, notamment un bovin, de préférence à la partie supérieure de la corne utérine, à proximité du réservoir spermatique, à l'aide d'un dispositif de transfert tel que défini ci-dessus, caractérisé par le fait qu'il comprend les étapes suivantes :
- introduire un matériel ou substance à but reproductif, thérapeutique ou diagnostique à transférer, dans la gaine intérieure par l'extrémité proximale de cette dernière, la gaine intérieure se trouvant dans la gaine extérieure ;
- introduire le piston dans la gaine intérieure et, dans le cas de l'utilisation d'une paillette de matériel ou substance à transférer, pousser la paillette jusqu'à arrêt de celle-ci par les moyens d'arrêt en translation puis transpercer la paillette par le piston, de telle sorte que celui-ci pousse le matériel ou substance à transférer vers l'extrémité distale de la gaine intérieure ;
- fixer la tête du piston à la navette primaire, cette dernière étant à l'extrémité proximale de la tige ;
- introduire la gaine extérieure dans le vagin d'un mammifère femelle, jusqu'à ce que l'extrémité distale de la gaine extérieure franchisse le col de l'utérus et s'oriente dans l'une des deux cornes utérines, en déchirant simultanément la chemise sanitaire lorsque celle-ci est présente ;
- déplacer l'ensemble navette en direction de la gaine extérieure, pour introduire la gaine intérieure dans la corne utérine jusqu'à ce que l'extrémité distale de la gaine intérieure soit dans la région de transfert ;
- continuer à déplacer l'ensemble navette en direction de la gaine extérieure tout en déplaçant la navette secondaire, vis-à-vis de la navette primaire, à l'opposé de la gaine extérieure, pour rétracter la gaine intérieure dans la gaine extérieure et ainsi permettre au matériel ou substance à transférer de sortir de l'extrémité distale de la gaine intérieure et d'être déposé dans la région de transfert ; et
- retirer le dispositif du mammifère femelle.

Pour mieux illustrer l'objet de la présente invention, on va en décrire ci-après, à titre indicatif et non limitatif, deux modes de réalisation particuliers avec référence aux dessins annexés.

Sur ces dessins :
- les Figures 1 et 2 sont des vues en perspective respectivement côté distal et côté proximal du dispositif d'insémination artificielle ou de transfert d'embryon selon un premier mode de réalisation de la présente invention, en position d'insertion dans l'animal ;
- la Figure 3 est une vue en coupe longitudinale du dispositif selon le premier mode de réalisation, avant la position d'insertion dans l'animal, les gaines extérieure et intérieure ayant été mises en place sur l'ensemble corps, avec vue de détail des extrémités distales des gaines extérieure et intérieure ;
- la Figure 4 est une vue en coupe longitudinale du dispositif selon le premier mode de réalisation, avant la position d'insertion dans l'animal, une paillette de semence ayant été insérée dans la gaine intérieure et un piston ayant été inséré dans la gaine intérieure après la paillette, avec vue de détail de l'épaulement d'arrêt et du rétrécissement de centrage de la paillette ;
- la Figure 5 est une vue en coupe longitudinale du dispositif selon le premier mode de réalisation, dans sa position d'insertion dans l'animal telle que représentée Figures 1 et 2, le piston ayant poussé le contenu de la paillette de semence vers l'extrémité distale du dispositif, avec vue de détail analogue à celle de la Figure 4 ;
- la Figure 6 est une vue en coupe longitudinale du dispositif selon le premier mode de réalisation, dans sa position après dépôt de la semence dans l'animal ;
- la Figure 7 est une vue de profil du piston selon une variante de réalisation ;
- la Figure 8 est une vue en perspective de l'extrémité distale de la gaine extérieure selon une variante de réalisation ; et
- la Figure 9 est une vue en coupe longitudinale de la partie distale du dispositif selon un second mode de réalisation particulier de la présente invention.

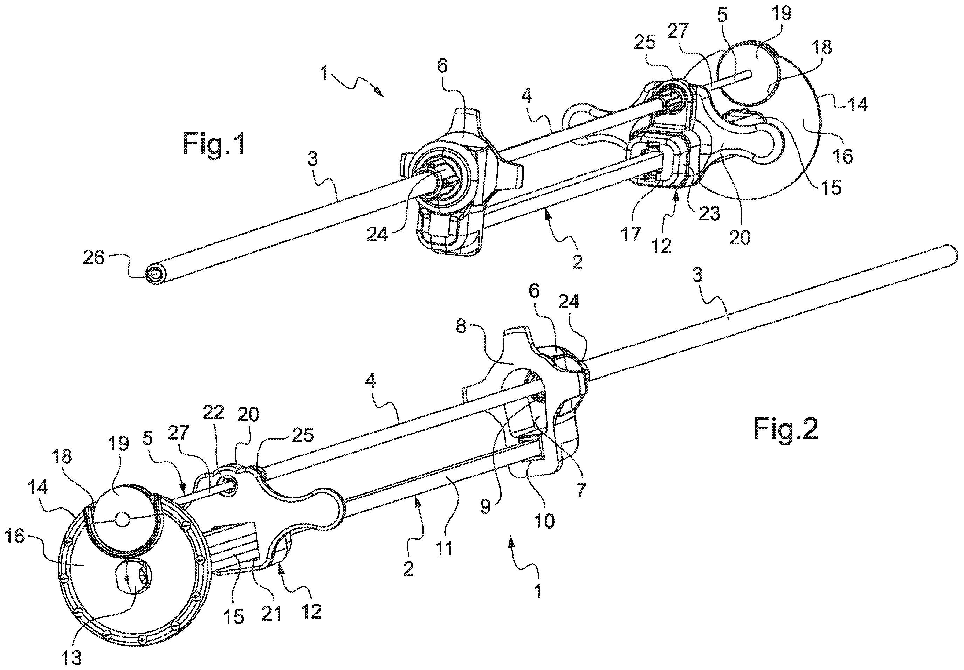

Si l'on se réfère aux Figures 1 et 2, on peut voir que l'on y a représenté un dispositif de transfert 1 selon la présente invention, représenté dans sa position d'insertion dans l'animal, lequel comprend un ensemble corps 2, une gaine extérieure 3, une gaine intérieure 4 et un piston 5.

Dans l'exemple représenté, le dispositif 1 est utilisé avec une paillette de semence et constitue ainsi un dispositif d'insémination artificielle. Ce dispositif 1 peut toutefois être utilisé pour le transfert d'un autre matériel ou substance à but reproductif, thérapeutique ou diagnostique.

L'ensemble corps 2 comprend un nez 6 sous la forme d'un corps sensiblement parallélépipédique, ayant un renfoncement 7 sur l'une de ses deux plus grandes faces 8 au fond duquel est formé un trou débouchant 9, ladite face 8 étant orientée vers le côté proximal du dispositif, et ayant un trou borgne 10 formé sur cette même grande face 8 et dans lequel une tige 11 est fixée. L'axe de la tige 11 est parallèle et coplanaire à l'axe de la gaine extérieure 3.

L'ensemble corps 2 comprend également un ensemble navette 12 monté coulissant sur ladite tige 11. La tige 11 est de section rectangulaire, ce par quoi l'ensemble navette 12 ne peut pas pivoter selon l'axe longitudinal de la tige 11.

La tige 11 comprend en outre une butée en translation 13 à l'extrémité proximale de la tige 11, afin d'éviter que l'ensemble navette 12 ne se détache de la tige 11.

L'ensemble navette 12 comprend une navette primaire 14 formée d'une partie longitudinale de section transversale rectangulaire 15, et d'une partie proximale 16 en forme de disque, et présente un trou traversant longitudinal 17 dont la section transversale correspond sensiblement à la section transversale de la tige 11. La partie proximale 16 en forme de disque comprend une encoche 18 recevant la tête 19 du piston 5.

L'ensemble navette 12 comprend également une navette secondaire 20 montée coulissante sur la partie longitudinale 15 de la navette primaire 14, par l'intermédiaire d'un premier trou traversant 21 dont la section transversale correspond sensiblement à la section transversale externe de la partie longitudinale 15 de la navette primaire 14, ce par quoi la navette secondaire 20 ne peut pas pivoter selon l'axe longitudinal de la navette primaire 14.

La navette secondaire 20 comprend en outre un second trou traversant 22 dans lequel passe le piston 5.

L'ensemble navette 12 comprend également une bague de blocage 23 fixée à l'extrémité distale de la navette primaire 14 et limitant le mouvement de coulissement de la navette secondaire 20 par rapport à la navette primaire 14.

La gaine extérieure 3 est reliée au corps 6 de l'ensemble corps 2 par un premier raccord Luer 24 au niveau du trou traversant 9 du nez 6, de telle sorte que la gaine extérieure 3 et ledit trou traversant 9 du nez 6 sont en communication entre eux.

La gaine intérieure 4 est insérée dans la gaine extérieure 3 et reliée par un raccord Luer 25 à la navette secondaire 20 au niveau du second trou traversant 22 de celle-ci, de telle sorte que la gaine intérieure 4 et ledit second trou traversant 22 sont en communication entre eux. La gaine intérieure 4 comprend en outre un embout d'extrémité 26.

Le piston 5 comprend un corps de piston 27 introduit dans la gaine intérieure 4. Le piston 5 est solidaire, dans cette position d'insertion dans l'animal, de la navette primaire 14, de telle sorte qu'un mouvement de translation de la navette primaire 14 permet de déplacer conjointement le piston 5 en translation.

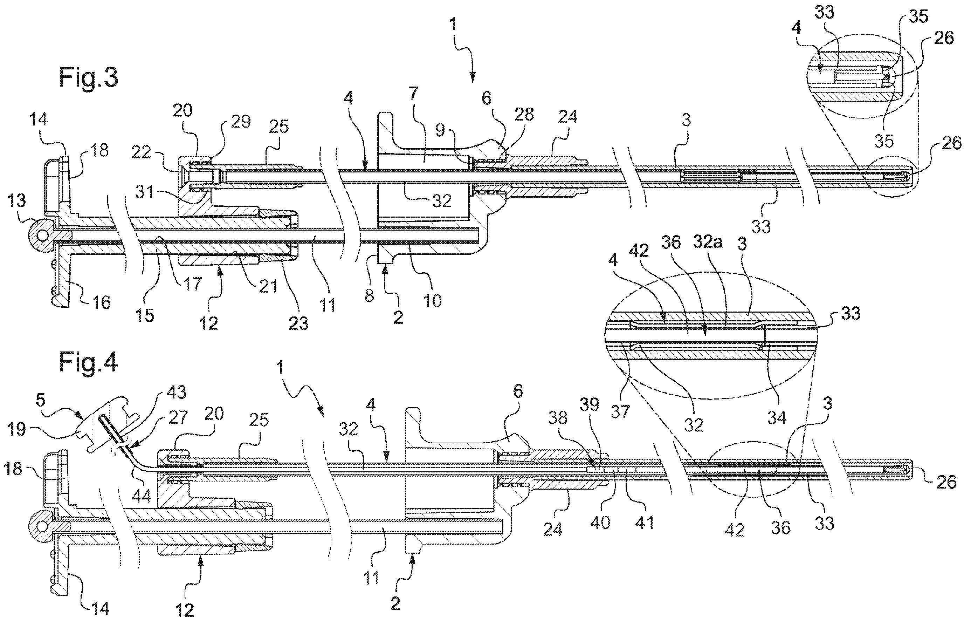

On va maintenant décrire plus en détail le fonctionnement du dispositif selon le mode de réalisation particulier, avec référence aux Figures 3 à 6.

Avant utilisation, un opérateur se saisit du dispositif 1 qui comprend alors l'ensemble corps 2 et dispose celui-ci sur un gabarit (non représenté). L'opérateur dispose des gaines extérieure 3 et intérieure 4 enfermées dans une chemise sanitaire extérieure (non représentée), qui entoure la surface longitudinale circonférentielle de la gaine extérieure 3 et également son extrémité distale. La chemise sanitaire comprenant les gaines 3, 4 est fixée sur le nez 6 et permet de maintenir le dispositif 1 propre jusqu'à son entrée dans le col utérin. La chemise sanitaire pourra par exemple être réalisée en matière plastique, telle que le polyéthylène.

Après avoir fait passer les gaines extérieure 3 et intérieure 4 à travers le trou traversant 9, depuis le côté distal du nez 6, la chemise sanitaire étant ensuite attachée sur une dent portée par le nez 6, on visse sur l'ensemble corps 2 le raccord Luer 24 qui relie la gaine extérieure 3 à l'ensemble corps 2, la partie distale dudit raccord Luer 24 présentant un filetage 28 et le trou traversant 9 du corps 6 présentant un taraudage correspondant.

Ensuite, l'opérateur relie la gaine intérieure 4 à la navette secondaire 20 par vissage du raccord Luer 25 cette dernière, la partie distale dudit raccord Luer 25 présentant un filetage 29 et le côté distal de la navette secondaire 30 présentant un renfoncement 31 concentrique au trou traversant 22 et comprenant un taraudage correspondant au filetage 29 sur la paroi latérale dudit renfoncement 31, afin d'obtenir le dispositif dans la configuration représentée sur la Figure 3.

Comme on peut mieux le voir sur la Figure 3, la gaine intérieure 4 est en fait réalisée en deux parties, à savoir une partie proximale 32 et une partie distale 33, la partie distale 33, destinée à s'étendre dans la corne utérine d'un animal, étant souple afin de ne pas blesser l'animal, tandis que la partie proximale 32, qui reste à l'intérieur de la gaine extérieure 3, peut être en une matière moins souple. La partie distale 33 pourra ainsi être réalisée en mélange de polyéthylène basse densité et de polyéthylène haute densité, tandis que la partie proximale 32 pourra être réalisée en polycarbonate.

Comme on peut le voir sur la vue de détail de la Figure 3, l'embout 26 qui ferme l'extrémité distale de la gaine intérieure 4 comprend deux orifices de sortie 35, ouvertures de transfert, disposés perpendiculairement à l'axe longitudinal de la gaine intérieure 4.

Ensuite, comme on peut le voir sur la Figure 4, l'opérateur insère dans la gaine intérieure 4 une paillette de semence 36, après préparation de cette dernière comme cela est bien connu (décongélation, etc.), puis insère le piston 5 dans ladite gaine intérieure 4 et pousse le piston 5 de telle sorte que la paillette 36 vient en butée contre des moyens d'arrêt en translation de la paillette 36.

Comme on peut le voir sur la vue de détail de la Figure 4, les moyens d'arrêt sont formés par un épaulement intérieur 34 formé par la face d'extrémité de la partie distale 33, côté proximal, du fait que la section de passage de la partie distale 33 est plus petite que la section de passage de la partie proximale 32, lesdites parties proximale 32 et distale 33 étant coaxiales. On peut également voir qu'est prévue, au voisinage de l'épaulement intérieur 34, une diminution de section, ou rétrécissement 32a, de la partie proximale 32, centrée sur l'axe longitudinal de la gaine intérieure 4 et qui sert à centrer la paillette 36 pour que l'espace intérieur de celle-ci soit directement en regard du canal interne de la partie distale 33, afin de permettre le passage de la semence de la paillette 36 à la partie distale 33.

La paillette 36, bien connue en soi, est formée d'un tube 37 dans lequel sont respectivement disposés, du côté proximal vers le côté distal de celle-ci, un bouchon 38 formé d'un premier morceau de coton 39, d'une partie en alcool polyvinylique (PVA) 40, et d'un second morceau de coton 41, et la semence à déposer 42.

La paillette 36 est fermée par scellage par fusion à son extrémité distale, et le bouchon 38 ferme l'autre extrémité.

Le corps de piston 27 du piston 5 est formé par une tige métallique 43 et un câble 44, l'extrémité proximale du câble 44 étant rendue solidaire de la tige 43 par tout moyen approprié, tel que par soudure et collage. Le diamètre du câble 44 est inférieur au diamètre intérieur du tube 37 de la paillette 36.

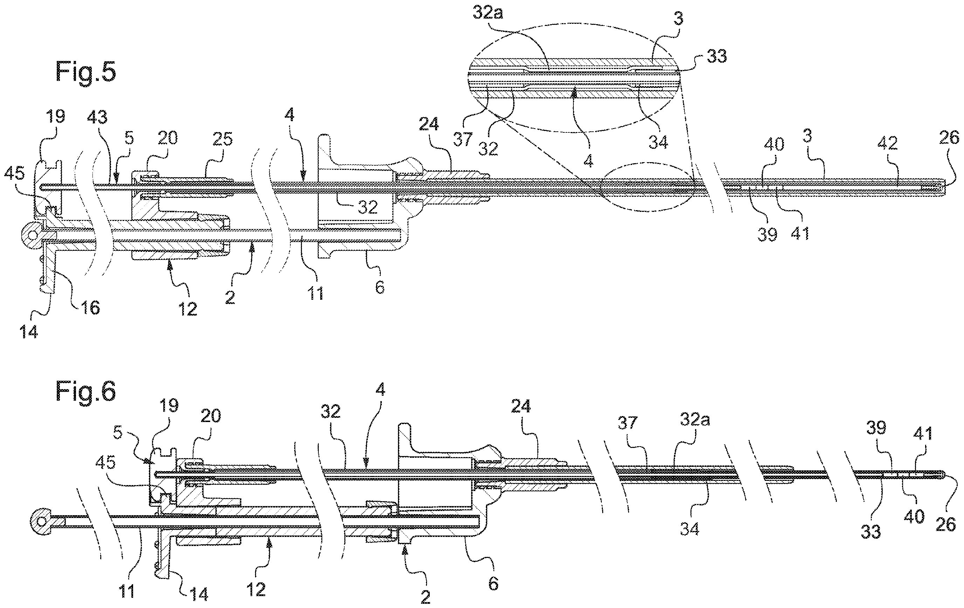

Ensuite, comme on peut le voir sur la Figure 5, l'opérateur pousse le piston 5 dans la direction distale de la gaine intérieure 4 par rapport à sa position de la Figure 4.

L'avancée du piston 5 a permis au câble 44, grâce à la rigidité suffisante offerte par la tige 43, de pousser le bouchon 38 de la paillette 36, et ainsi de pousser le contenu de celle-ci vers l'extrémité distale de la gaine intérieure 4, le tube 37 de la paillette 36 étant maintenu en position par les moyens d'arrêt 34.

Le piston 5 est avancé par l'opérateur jusqu'à une position dans laquelle il est disposé parallèlement à la tige 11 et dans laquelle la tête du piston 19 est insérée dans l'encoche 18 de la partie proximale 16 de la navette primaire 14.

Des moyens de blocage en position 45 du piston 5, par complémentarité de formes, sont prévus dans ladite encoche 18, afin de solidariser en translation la navette primaire 14 et le piston 5.

La longueur du piston 5 est choisie de telle sorte que, dans la position de la Figure 5, un espace est formé dans l'extrémité distale de la gaine intérieure 4, entre l'extrémité distale du piston 5 et l'embout d'extrémité 26 de la gaine intérieure 4, et de telle sorte que ledit espace peut contenir le contenu de la paillette 36 (bouchon 38 et semence 42) sans que la semence 42 ne s'écoule par les orifices 35 de l'embout 26.

On peut mentionner les caractéristiques suivantes :
- gaine extérieure : longueur de 443 mm, diamètre extérieur de 5,9 mm et diamètre intérieur de 3,8 mm ;
- gaine intérieure : longueur de 799,5 mm, diamètre extérieur de 3,65 mm et diamètre intérieur de 2,65 mm pour la partie proximale en amont du rétrécissement, diamètre intérieur de 2,5 mm au niveau du rétrécissement, et diamètre extérieur de 2,5 mm et diamètre intérieur de 1,6 mm pour la partie distale ;
- piston : longueur de la tige métallique de 386 mm, longueur du câble de 390 mm, diamètre du câble de 1,25 mm.

On comprend, d'après la Figure 5, que les mouvements des différents éléments de l'ensemble navette 12 permettent d'obtenir, à partir de la position que l'on peut voir sur ladite Figure 5, différents mouvements des éléments du dispositif 1, à savoir :
- un mouvement de l'ensemble navette 12 vers le côté distal du dispositif 1 permet de déplacer la gaine intérieure 4, son contenu et le piston 5 vers le côté distal du dispositif 1, pour amener la gaine intérieure 4, avec le câble 44 à l'intérieur de celle-ci, à sortir par l'extrémité distale de la gaine extérieure 3 ; et
- un mouvement de la navette secondaire vers le côté proximal du dispositif 1 permet de déplacer la gaine intérieure 4 seule vers le côté proximal du dispositif, et de faire sortir la semence 42 par les orifices 35, le piston 5 restant immobile, l'action de déplacement de la gaine intérieure 4 sur le piston 5 fixe s'exerçant alors comme une poussée du piston 5 vers le côté distal.

Ainsi, afin de déposer la semence 42, après avoir identifié le côté ovulatoire, l'opérateur introduit l'extrémité distale du dispositif 1, dans la position de la Figure 5, dans le vagin de l'animal.

Le diamètre et la matière de la gaine extérieure 3 sont choisis pour lui permettre de passer le col de l'utérus de l'animal avec facilité et sans blesser l'animal, et sa longueur choisie pour que son extrémité distale puisse atteindre le début de l'une des cornes utérines de l'animal. L'opérateur déchire la chemise sanitaire à l'entrée du col de l'utérus.

Une fois que l'extrémité distale de la gaine extérieure 3 est en position au début d'une corne utérine, l'opérateur pousse d'une main l'ensemble navette 12 de façon à faire sortir la gaine intérieure 4 jusqu'à ce que l'extrémité distale de la gaine intérieure 4 atteigne la région de transfert adéquate, à savoir la partie supérieure de la corne utérine, à proximité du réservoir spermatique, l'opérateur guidant et contrôlant de son autre main, en passant par le rectum de l'animal, le déplacement de la gaine intérieure 4 dans la corne utérine.

La navette secondaire 20 n'est à ce moment pas déplacée par rapport à la navette primaire 14, de sorte qu'aucun mouvement relatif entre la gaine intérieure 4 et le piston 5 n'est produit.

On souligne ici que, dans cette phase, le bouchon 38 entoure déjà l'extrémité distale du câble 45 et protège ainsi l'animal de l'extrémité du piston 5 au cours de la progression de la gaine souple 4 et du piston 5 dans la corne utérine.

L'opérateur tire ensuite la navette secondaire 20 vers le côté proximal du dispositif 1 par sa main libre, avec son pouce en appui contre la partie proximale 16 en forme de disque et ses autres doigts qui appuient sur la navette secondaire 20, ou inversement, à la manière d'une seringue. La gaine intérieure 4 est ainsi rétractée sur le piston 5 et la semence 42 est poussée hors de la gaine intérieure 4 par les orifices 35 pour être déposée dans l'animal.

A la fin de l'opération de transfert, le dispositif 1 peut se trouver, en fonction de l'anatomie de l'animal, dans la position représentée sur la Figure 6, sur laquelle on peut voir que l'ensemble navette 12 a été déplacé vers le côté distal du dispositif 1 jusqu'à venir en butée contre le corps 6 de l'ensemble corps 2 et que la navette secondaire 20 a été déplacée vers le côté proximal de la navette primaire 14 jusqu'à venir en butée contre celui-ci.

Dans cette position, on peut voir que la partie distale 33 de la gaine intérieure 4 se situe, pour sa plus grande part, hors de la gaine extérieure 3, et que la semence 42 a bien été poussée hors du dispositif 1 par les deux orifices 35, le bouchon 38 de la paillette 36 restant dans l'embout distal 26 de la gaine intérieure 4.

On constate donc que le dispositif selon la présente invention permet de déposer la quasi-totalité du matériel ou substance à transférer dans la région adéquate, pour un meilleur effet du transfert, tel qu'un meilleur taux de succès de l'insémination artificielle ou du transfert d'embryon, sans risquer de blesser l'animal.

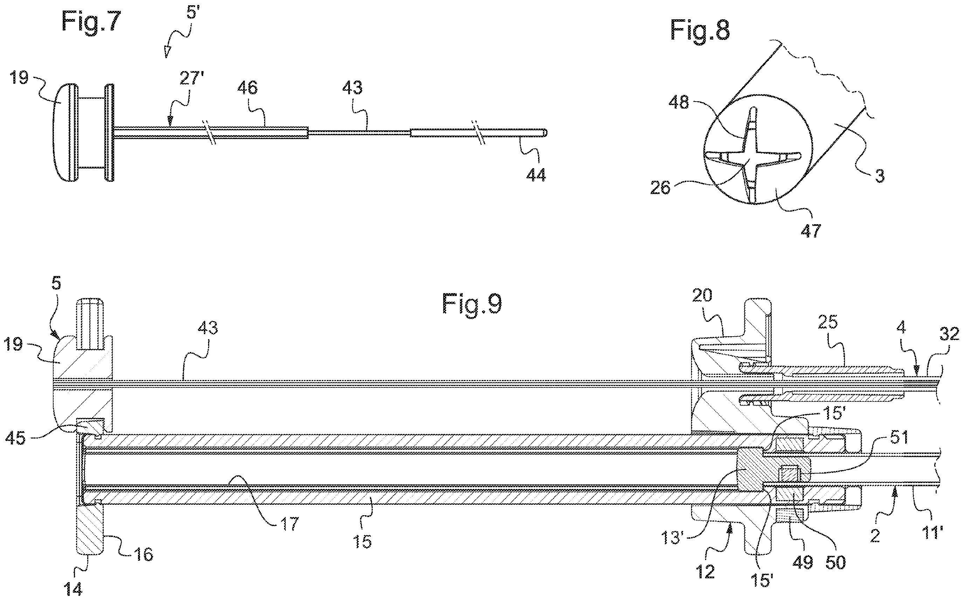

Si l'on se réfère maintenant à la Figure 7, on peut voir que l'on y a représenté une variante du piston, désigné par le chiffre de référence 5', dont le corps 27' comprend une partie rigide 46, ici formée par un jonc en inox à l'extrémité distale duquel est fixée l'extrémité proximale de la tige 43. Le câble 44 s'étend à partir de l'extrémité distale de la tige 43. Le diamètre de la tige 43 est choisi pour être inférieur ou égal au diamètre du câble 44, de sorte que la tige 43 d'une part peut également traverser la paillette 36 et, d'autre part, est plus souple que dans le premier mode de réalisation et peut donc être également étendue dans la partie distale 33 de la gaine intérieure 4. Le jonc constitue un moyen de rigidification de la partie proximale du piston 5' et ainsi d'assurer la poussée du piston 5' dans la gaine intérieure 4. En d'autres termes, le corps 27' du piston 5' comprend une partie distale souple, formée par le câble 44, une partie intermédiaire également souple, formée par la tige 43 et qui traverse la paillette 36, et une partie proximale rigide 46.

Dans cette variante :
- la longueur exposée de la tige métallique 43 est de 120,3 mm et son diamètre de 1,2 mm ;
- la longueur du jonc 46 est de 332,3 mm, son diamètre externe est de 2 mm ; et
- la longueur du câble 44 est de 265 mm et son diamètre est de 1,25 mm.

Pour les gaines, on pourra utiliser une gaine extérieure d'une longueur de 423 mm et une gaine intérieure d'une longueur de 726 mm, les autres caractéristiques des gaines mentionnées en rapport avec le premier mode de réalisation restant inchangées.

Cet agencement particulier du piston 5' permet de rigidifier ce dernier, ce qui évite qu'il se plie et facilite la décharge de la semence.

Bien entendu, pour un résultat analogue à la variante décrite ci-dessus, dans le premier mode de réalisation on pourrait prévoir une tige métallique 43 de plus petit diamètre, pour qu'elle puisse traverser la paillette, mais dont la partie proximale serait rigidifiée, par exemple en étant noyée dans un tube rigide. Si l'on se réfère maintenant à la Figure 8, on peut voir que l'extrémité distale de la gaine extérieure 3 comprend, selon une variante de réalisation, un embout 47 en forme d'ogive et en matériau souple, tel qu'un élastomère, percé d'une ouverture en croix 48. Le diamètre de l'embout est strictement identique à celui de la gaine extérieure 3. Une telle forme permet d'améliorer encore davantage le caractère atraumatisant du dispositif et de protéger davantage l'embout d'extrémité 26 de la gaine intérieure 4.

Si l'on se réfère enfin à la Figure 9, représentant un second mode de réalisation particulier de la présente invention, on peut voir que la navette secondaire 20 est munie d'un premier aimant 49, la partie longitudinale 15 de la navette primaire 14 est munie, à son extrémité distale, d'un deuxième aimant 50, et la tige 11' comprend une butée 13' munie d'un troisième aimant 51.

La tige 11' est plus courte que la tige 11, et la butée 13' est apte à entrer dans la partie longitudinale 15. La butée 13' vient en butée contre un épaulement 15' formé dans le trou traversant 17 de la partie longitudinale 15. Ceci permet d'obtenir un dispositif encore plus compact.

Les aimants 49, 50, 51 sont agencés avec leurs pôles d'une manière telle que lorsqu'ils ont leurs centres sensiblement alignés sur une même droite, c'est-à-dire en position avant l'insertion dans l'animal, comme représenté sur la Figure 9, le deuxième aimant 50 attire les premier et troisième aimants 49 et 51.

Ainsi, les éléments du dispositif sont maintenus en place lorsque, par exemple, l'opérateur manipule l'appareil avant l'insémination artificielle.

Ceci permet d'éviter un déplacement involontaire des éléments du dispositif, lequel déplacement accidentel pourrait conduire à l'éjection du contenu de la paillette 33 hors du dispositif.

De plus, le maintien temporaire en position des navettes primaire 14 et secondaire 20 par des aimants procure une douceur à l'actionnement de ces dernières : les navettes 14 et 20 ne sont pas déplacées de manière brusque au début de leur déplacement.

Il est bien entendu que le mode de réalisation ci-dessus de la présente invention a été donné à titre indicatif et non limitatif et que des modifications pourront y être apportées sans que l'on s'écarte pour autant du cadre de la présente invention.

Par exemple, on pourra modifier la structure de l'ensemble corps, et par exemple ne pas utiliser un nez comme décrit ci-dessus, ou ajouter une graduation sur la tige 11, afin de permettre à l'opérateur de connaître la distance sur laquelle le matériel de reproduction a été déposé, dans le cas où l'ensemble navette 12 a été déplacé dans la direction proximale du dispositif 1 au cours de l'opération de transfert en lui-même.

## Revendications

1. Dispositif (1) de transfert atraumatique de matériel ou substance à but reproductif, thérapeutique ou diagnostique dans un mammifère femelle, notamment un bovin, permettant un transfert dans une région, dite de transfert, d'une corne utérine d'un mammifère femelle, comprenant :
- un ensemble corps (2) ;
- une gaine extérieure (3) ayant une extrémité proximale ouverte et une extrémité distale ouverte, la gaine extérieure (3) étant solidaire de l'ensemble corps (2) et apte à être introduite dans le vagin du mammifère femelle et franchir le col de l'utérus du mammifère femelle ;
- une gaine intérieure (4) ayant une extrémité proximale ouverte, une extrémité distale dans laquelle est ménagée au moins une ouverture (35) dite de transfert, et un canal interne s'étendant entre les extrémités proximale et distale de la gaine intérieure (4) et apte à recevoir un matériel, ou une substance, à but reproductif, thérapeutique ou diagnostique à transférer, la gaine intérieure (4) étant reçue de manière coulissante dans la gaine extérieure (3) et étant souple de façon à être apte à être étendue, à partir de l'extrémité distale de la gaine extérieure (3), dans la corne utérine du mammifère femelle jusqu'à ce que l'extrémité distale de la gaine intérieure (4) se situe dans la région de transfert ; et
- un piston (5 ; 5') destiné, après mise en place du matériel ou substance à transférer dans la gaine intérieure (4), à être introduit dans la gaine intérieure (4) de manière à pouvoir coulisser dans celle-ci et à conduire, par un mouvement de translation relatif entre le piston (5 ; 5') et la gaine intérieure (4), le matériel ou substance à transférer vers l'extrémité distale de la gaine intérieure (4), par laquelle le matériel ou substance à transférer sera déposé dans la région de transfert,
dispositif (1) **caractérisé par le fait qu'**une partie, dite distale, du piston (5 ; 5') est souple et apte à s'étendre dans la partie, dite distale (33), de la gaine intérieure (4) qui est destinée à s'étendre dans la corne utérine et **par le fait que** le dispositif (1) comprend des moyens de déplacement conjoint de la gaine intérieure (4) et du piston (5 ; 5') jusqu'à la région de transfert, avec ladite partie distale (33) du piston (5 ; 5') se situant à l'intérieur de ladite partie distale de la gaine intérieure (4), et des moyens de rétractation de la gaine intérieure (4) sur le piston (5 ; 5') au niveau de la région de transfert, de façon à faire sortir le matériel ou substance à transférer par l'extrémité distale de la gaine intérieure (4), sous la poussée du piston (5 ; 5').

2. Dispositif (1) selon la revendication 1, **caractérisé par le fait que** l'ensemble corps (2) comprend une tige (11 ; 11') s'étendant dans la direction à l'opposé de la gaine extérieure (3) et sur laquelle est monté coulissant un ensemble navette (12) qui comprend une navette primaire (14), montée coulissante sur ladite tige (11 ; 11') et à laquelle le piston (5 ; 5') est fixé, et une navette secondaire (20), montée coulissante sur ladite navette primaire (14) et à laquelle l'extrémité proximale de la gaine intérieure (4) est fixée, la navette primaire (14) et la navette secondaire (20) constituant lesdits moyens de déplacement conjoint et lesdits moyens de rétractation.

3. Dispositif (1) selon l'une des revendications 1 ou 2, **caractérisé par le fait que** la gaine intérieure (4) comprend une partie proximale (32), à l'intérieur de la gaine extérieure (3), et la partie distale souple (33), de préférence d'une longueur supérieure à 130 mm, de façon davantage préférée d'une longueur de 255,5 mm, destinée à s'étendre dans la corne utérine, la partie proximale (32) étant moins souple que la partie distale (33).

4. Dispositif (1) selon l'une des revendications 1 à 3, approprié au transfert de matériel ou substance à but reproductif, thérapeutique ou diagnostique contenu(e) dans l'espace intérieur, de section transversale donnée, d'une paillette (36), **caractérisé par le fait que** le canal interne de la gaine intérieure (4) est apte à recevoir une telle paillette (36), le piston (5 ; 5') est apte à traverser la paillette (36) pour en faire sortir le matériel ou substance à transférer (42) et la gaine intérieure (4) comprend des moyens d'arrêt en translation de la paillette (36) dans la direction de l'extrémité distale, les moyens d'arrêt en translation de la paillette (36) étant notamment formés par un épaulement intérieur (34) dans la gaine intérieure (4), le cas échéant situé à la jonction entre les parties proximale (32) et distale (33) de la gaine intérieure (4), et, de préférence, centré sur l'axe de la gaine intérieure (4).

5. Dispositif (1) selon la revendication 4, les moyens d'arrêt en translation étant formés par un épaulement intérieur (34) et pour une utilisation avec une paillette (36) dont l'espace intérieur a une section transversale égale à la section de passage du canal interne au niveau de l'épaulement intérieur (34), **caractérisé par le fait que** sont prévus des moyens de centrage de la paillette (36) sur l'axe de la gaine intérieure (4) lorsque la paillette (36) est mise en butée contre les moyens d'arrêt en translation, lesdits moyens de centrage étant notamment formés par un rétrécissement (32a) de la section de passage du canal interne de la gaine intérieure (4) au voisinage de l'épaulement (34), côté extrémité proximale de la gaine intérieure (4), le rétrécissement (32a) étant centré sur l'axe de la gaine intérieure (4).

6. Dispositif (1) selon l'une des revendications 1 à 5, **caractérisé par le fait que** l'ensemble corps (2) comprend un nez (6) dans lequel sont ménagés un passage traversant (9), à partir d'une extrémité duquel s'étend la gaine extérieure (3), sur un premier côté du nez (6), et un trou débouchant (10) sur un second coté (8) du nez (6), opposé audit premier côté, et à partir duquel s'étend la tige (11 ; 11') de l'ensemble corps (2), l'axe du passage traversant (9) et l'axe du trou (10) étant parallèles et coplanaires.

7. Dispositif (1) selon l'une des revendications 1 à 6, **caractérisé par le fait que** la navette secondaire (20) comprend un passage traversant (22) pour le piston (5 ; 5') et que la gaine intérieure (4) est reliée à la navette secondaire (20) par un raccord (25), notamment de type Luer.

8. Dispositif (1) selon l'une des revendications 1 à 7, **caractérisé par le fait que** les extrémités distales de la gaine extérieure (3) et de la gaine intérieure (4) sont exemptes d'arêtes et bords tranchants, lesdites extrémités distales étant, de préférence, bombées, de façon à faciliter la progression des gaines extérieure (3) et intérieure (4) dans le mammifère sans blesser ce dernier.

9. Dispositif (1) selon l'une des revendications 1 à 8, **caractérisé par le fait que** l'extrémité distale de la gaine intérieure (4) est formée par un embout (26) ayant deux sorties latérales (35) permettant de déposer le matériel ou substance à transférer dans la région de transfert, en particulier de la semence en direction des cryptes spermatiques, ledit embout (26) présentant, de préférence, une forme bombée à son extrémité.

10. Dispositif (1) selon l'une des revendications 1 à 8, **caractérisé par le fait que** l'extrémité distale de la gaine extérieure (3) est formée par un embout (47) en matière souple, en forme d'ogive et percé d'une ouverture en croix (48).

11. Dispositif (1) selon l'une des revendications 1 à 10, **caractérisé par le fait que** le piston (5) est formé par une combinaison d'une tige (43), notamment métallique, et d'un câble (44), respectivement côté proximal et côté distal du dispositif (1), le câble (44) formant la partie distale du piston (5) destinée à s'étendre dans la partie de la gaine intérieure (4) destinée à s'étendre dans la corne utérine du mammifère.

12. Dispositif (1) selon la revendication 4, **caractérisé par le fait que** le piston (5') est formé par un jonc rigide (46), formant la partie proximale du piston (5') par laquelle il est, le cas échéant, fixé à la navette primaire (14), par une tige (43) souple et apte à traverser la paillette (36), la tige (43), qui est notamment métallique, s'étendant à partir d'une extrémité distale du jonc (46), et par un câble (44) s'étendant à partir d'une extrémité distale de la tige (43), le câble (44) et la région d'extrémité distale de la tige (43) formant la partie distale du piston (5') destinée à s'étendre dans la partie de la gaine intérieure (4) destinée à s'étendre dans la corne utérine du mammifère.

13. Dispositif (1) selon l'une des revendications 1 à 12, **caractérisé par le fait que** la longueur du piston (5 ; 5') est sensiblement égale à celle de la gaine intérieure (4).

14. Dispositif (1) selon l'une des revendications 2 à 13, **caractérisé par le fait que** la navette secondaire (20) est munie d'un premier aimant (49), la navette primaire (14) est munie, dans sa région d'extrémité distale, d'un deuxième aimant (50), et la tige (11') est munie d'un troisième aimant (49) dans sa région d'extrémité proximale, les aimants (49, 50, 51) étant configurés pour être aptes à être alignés les uns avec les autres dans une position donnée des navettes primaire (14) et secondaire (20) et s'attirer les unes les autres de façon à maintenir en position les navettes primaire (14) et secondaire (20) par rapport à la tige (11').

15. Dispositif selon l'une des revendications 2 à 14, **caractérisé par le fait que** la tige (11 ; 11') porte à son extrémité distale une butée (13 ; 13'), de préférence une butée (13') reçue dans un trou traversant (17) de la navette primaire (14), par lequel la navette primaire (14) est montée coulissante sur la tige (11), le trou traversant (17) comprenant, de préférence à son extrémité distale, un épaulement (15') présentant une surface d'arrêt pour ladite butée (13').

## Patentansprüche

1. Vorrichtung (1) zur atraumatischen Übertragung von Material oder Substanz zu reproduktiven, therapeutischen oder diagnostischen Zwecken bei einem weiblichen Säugetier, insbesondere einem Rind, die in eine Region, bezeichnet als Übertragungsregion, die Übertragung eines Uterushorns eines weiblichen Säugetiers ermöglicht, umfassend:
- eine Körpereinheit (2);
- eine äußere Umhüllung (3), aufweisend ein offenes proximales Ende und ein offenes distales Ende, wobei die äußere Umhüllung (3) fest mit der Körpereinheit (2) verbunden und ausgelegt ist, um in die Scheide des weiblichen Säugetiers eingeführt zu werden und den Gebärmutterhals des weiblichen Säugetiers zu überwinden;
- eine innere Umhüllung (4), aufweisend ein offenes proximales Ende und ein distales Ende, in der mindestens eine Öffnung (35), bezeichnet als Übertragungsöffnung, angebracht ist, und einen internen Kanal, der sich zwischen dem proximalen und dem distalen Ende der inneren Umhüllung (4) erstreckt und ausgelegt ist, um ein Material oder eine Substanz zu reproduktiven, therapeutischen oder diagnostischen Zwecken, die übertragen werden soll, aufzunehmen, wobei die innere Umhüllung (4) auf gleitende Weise in der äußeren Umhüllung (3) aufgenommen ist und flexibel ist, um dazu ausgelegt zu sein, vom distalen Ende der äußeren Umhüllung (3) im Uterushorn des weiblichen Säugetiers ausgedehnt zu werden, bis sich das distale Ende der inneren Umhüllung (4) in der Übertragungsregion befindet; und
- einen Kolben (5; 5'), der, nach der Anordnung des Materials oder der Substanz, die übertragen werden soll, in der inneren Umhüllung (4), dazu vorgesehen ist, um in die innere Umhüllung (4) eingeführt zu werden, um in derselben gleiten zu können und um, durch eine relative Translationsbewegung zwischen dem Kolben (5; 5') und der inneren Umhüllung (4), das Material oder die Substanz, die übertragen werden soll, hin zum distalen Ende der inneren Umhüllung (4) zu übertragen, durch das das Material oder die Substanz, die übertragen werden soll, in der Übertragungsregion abgelegt wird,
Vorrichtung (1), **dadurch gekennzeichnet, dass** ein Teil, bezeichnet als distaler Teil, des Kolbens (5; 5') flexibel und ausgelegt ist, um sich im Teil, bezeichnet als distaler Teil (33), der inneren Umhüllung (4) zu erstrecken, der dazu vorgesehen ist, sich im Uterushorn zu erstrecken, und dadurch, dass die Vorrichtung (1) Mittel zur gemeinsamen Verschiebung der inneren Umhüllung (4) und des Kolbens (5; 5') bis in die Übertragungsregion umfasst, wobei sich der distale Teil (33) des Kolbens (5; 5') im Inneren des distalen Teils der inneren Umhüllung (4) befindet, und Mittel zum Rückzug der inneren Umhüllung (4) auf dem Kolben (5; 5') auf der Ebene der Übertragungsregion, um das Material oder die Substanz, die übertragen werden soll, durch das distale Ende der inneren Umhüllung (4) unter dem Schub des Kolbens (5; 5') austreten zu lassen.

2. Vorrichtung (1) nach Anspruch 1, **dadurch gekennzeichnet, dass** die Körpereinheit (2) einen Stift (11; 11') umfasst, der sich in der Richtung gegenüber der äußeren Umhüllung (3) erstreckt, und auf dem gleitend eine Shuttleeinheit (12) montiert ist, die ein primäres Shuttle (14) umfasst, das gleitend auf den Stift (11; 11') montiert ist, und an das der Kolben (5; 5') fixiert ist, und ein sekundäres Shuttle (20), das gleitend auf dem primären Shuttle (14) montiert ist, und an das das proximale Ende der inneren Umhüllung (4) fixiert ist, wobei das primäre Shuttle (14) und das sekundäre Shuttle (20) die Mittel zur gemeinsamen Verschiebung und die Mittel zum Rückzug darstellen.

3. Vorrichtung (1) nach einem der Ansprüche 1 oder 2, **dadurch gekennzeichnet, dass** die innere Umhüllung (4) einen proximalen Teil (32) im Inneren des äußeren Umhüllung (3) und den flexiblen distalen Teil (33) umfasst, vorzugsweise mit einer Länge von mehr als 130 mm, noch bevorzugter mit einer Länge von 255,5 mm, der dazu vorgesehen ist, um sich im Uterushorn zu erstrecken, wobei der proximale Teil (32) weniger flexibel als der distale Teil (33) ist.

4. Vorrichtung (1) nach einem der Ansprüche 1 bis 3, die geeignet ist für die Übertragung von Material oder Substanz mit reproduktiven, therapeutischen oder diagnostischen Zwecken, enthalten im inneren Raum mit gegebenem Querschnitt einer Paillette (36), **dadurch gekennzeichnet, dass** der interne Kanal der inneren Umhüllung (4) ausgelegt ist, eine derartige Paillette (36) aufzunehmen, der Kolben (5; 5') ausgelegt ist, die Paillette (36) zu queren, um das Material oder die Substanz, die übertragen werden soll (42), davon austreten zu lassen, und die innere Umhüllung (4) Mittel zum Stopp in Translation der Paillette (36) in der Richtung des distalen Endes umfasst, wobei die Mittel zum Stopp in Translation der Paillette (36) insbesondere durch einen inneren Absatz (34) in der inneren Umhüllung (4) gebildet sind, der sich gegebenenfalls an der Verbindung zwischen dem proximalen (32) und distalen Teil (33) der inneren Umhüllung (4) befindet, und vorzugsweise zentriert auf der Achse der inneren Umhüllung (4).

5. Vorrichtung (1) nach Anspruch 4, wobei die Mittel zum Stopp in Translation durch einen inneren Absatz (34) und für eine Verwendung mit einer Paillette (36) gebildet sind, dessen innerer Raum einen Querschnitt aufweist, der gleich dem Durchgang des internen Kanals auf dem Niveau des inneren Absatzes (34) ist, **dadurch gekennzeichnet, dass** Mittel zur Zentrierung der Paillette (36) auf der Achse der inneren Umhüllung (4) vorgesehen sind, wenn die Paillette (36) gegen die Mittel zum Stopp in Translation in Anschlag gebracht wird, wobei die Mittel zur Zentrierung insbesondere durch eine Verengung (32a) des Durchgangs des internen Kanals der inneren Umhüllung (4) benachbart dem Absatz (34) auf der Seite des proximalen Endes der inneren Umhüllung (4) gebildet sind, wobei die Verengung (32a) auf der Achse der inneren Umhüllung (4) zentriert ist.

6. Vorrichtung (1) nach einem der Ansprüche 1 bis 5, **dadurch gekennzeichnet, dass** die Körpereinheit (2) eine Nase (6) umfasst, in der ein querender Durchgang (9), ausgehend von einem Ende, von dem sich die äußere Umhüllung (3) erstreckt, auf einer ersten Seite der Nase (6), und ein Loch (10) das auf einer zweiten Seite (8) der Nase (6), gegenüber der ersten Seite, mündet, angebracht sind, und ausgehend von dem sich der Stift (11; 11') der Körpereinheit (2) erstreckt, wobei die Achse (9) des querenden Durchgangs und die Achse des Lochs (10) parallel und koplanar sind.

7. Vorrichtung (1) nach einem der Ansprüche 1 bis 6, **dadurch gekennzeichnet, dass** das sekundäre Shuttle (20) einen querenden Durchgang (22) für den Kolben (5; 5') umfasst, und dass die innere Umhüllung (4) mit dem sekundären Shuttle (20) durch einen Anschluss (25), insbesondere vom Typ Luer, verbunden ist.

8. Vorrichtung (1) nach einem der Ansprüche 1 bis 7, **dadurch gekennzeichnet, dass** die distalen Enden der äußeren Umhüllung (3) und der inneren Umhüllung (4) frei von Kanten und scharfen Rändern sind, wobei die distalen Enden vorzugsweise gewölbt sind, um das Fortschreiten der äußeren (3) und inneren Umhüllung (4) im Säugetier zu erleichtern, ohne Letzteres zu verletzen.

9. Vorrichtung (1) nach einem der Ansprüche 1 bis 8, **dadurch gekennzeichnet, dass** das distale Ende der inneren Umhüllung (4) durch einen Stutzen (26) gebildet ist, aufweisend zwei seitliche Ausgänge (35), die ermöglichen, das Material oder die Substanz, die übertragen werden soll, in der Übertragungsregion abzulegen, insbesondere Samen in Richtung der Spermavertiefungen, wobei der Stutzen (26) vorzugsweise eine gewölbte Form an seinem Ende aufweist.

10. Vorrichtung (1) nach einem der Ansprüche 1 bis 8, **dadurch gekennzeichnet, dass** das distale Ende der äußeren Umhüllung (3) durch einen Stutzen (47) aus flexiblem Material in Form einer Ogive, durchbrochen von einer kreuzförmigen Öffnung (48), gebildet ist.

11. Vorrichtung (1) nach einem der Ansprüche 1 bis 10, **dadurch gekennzeichnet, dass** der Kolben (5) durch eine Kombination eines Stifts (43), insbesondere aus Metall, und eines Kabels (44) auf der proximalen Seite bzw. der distalen Seite der Vorrichtung (1) gebildet ist, wobei das Kabel (44) den distalen Teil des Kolbens (5) bildet, der dazu vorgesehen ist, sich im Teil der inneren Umhüllung (4) zu erstrecken, der dazu vorgesehen ist, sich im Uterushorn des Säugetiers zu erstrecken.

12. Vorrichtung (1) nach Anspruch 4, **dadurch gekennzeichnet, dass** der Kolben (5') durch einen starren Reif (46) gebildet ist, der den proximalen Teil des Kolbens (5') bildet, durch den er gegebenenfalls an das primäre Shuttle (14) fixiert ist, durch einen flexiblen Stift (43) und der ausgelegt ist, um die Paillette (36) zu queren, wobei sich der Stift (43), der insbesondere aus Metall ist, ausgehend von einem distalen Ende des Reifs (46) erstreckt, und durch ein Kabel (44), das sich ausgehend von einem distalen Ende des Stifts (43) erstreckt, wobei das Kabel (44) und die distale Endregion des Stifts (43) den distalen Teil des Kolbens (5') bilden, der dazu vorgesehen ist, sich im Teil der inneren Umhüllung (4) zu erstrecken, der dazu vorgesehen ist, sich im Uterushorn des Säugetiers zu erstrecken.

13. Vorrichtung (1) nach einem der Ansprüche 1 bis 12, **dadurch gekennzeichnet, dass** die Länge des Kolbens (5; 5') im Wesentlichen gleich derjenigen der inneren Umhüllung (4) ist.

14. Vorrichtung (1) nach einem der Ansprüche 2 bis 13, **dadurch gekennzeichnet, dass** das sekundäre Shuttle (20) mit einem ersten Magnet (49) ausgestattet ist, das primäre Shuttle (14) in seiner distalen Endregion mit einem zweiten Magnet (50) ausgestattet ist und der Stift (11') mit einem dritten Magnet (49) in seiner proximalen Endposition ausgestattet ist, wobei die Magneten (49, 50, 51) konfiguriert sind, um ausgelegt zu sein, um untereinander in einer gegebenen Position des primären Shuttles (14) und des sekundären Shuttles (20) ausgefluchtet zu sein und sich gegenseitig anzuziehen, um das primäre (14) und das sekundäre Shuttle (20) mit Bezug auf den Stift (11') in ihrer Position zu halten.

15. Vorrichtung (1) nach einem der Ansprüche 2 bis 14, **dadurch gekennzeichnet, dass** der Stift (11; 11') an seinem distalen Ende einen Anschlag (13; 13') trägt, vorzugsweise einen Anschlag (13'), der in einem Loch aufgenommen ist, das das primäre Shuttle (14) quert (17), durch das das primäre Shuttle (14) gleitend auf dem Stift (11') montiert ist, wobei das querende Loch (17), vorzugsweise an seinem distalen Ende, einen Absatz (15') umfasst, der eine Stoppfläche für den Anschlag (13') darstellt.

## Claims

1. A device (1) for the atraumatic transfer of a material or substance with a reproductive, therapeutic or diagnostic purpose into a female mammal, such as a bovine, allowing a transfer into a region, so-called transfer region, of an uterine horn of a female mammal, the device comprising:
- a body assembly (2);
- an outer sheath (3) having an open proximal end and an open distal end, the outer sheath (3) being integral with the body assembly (2) and able to be inserted into the vagina of the female mammal and to pass the uterine cervix of the female mammal;
- an inner sheath (4) having an open proximal end, a distal end in which at least one opening (35), so-called transfer opening, is provided, and an inner channel extending between the proximal and distal ends of the inner sheath (4) and able to receive a material, or a substance, with a reproductive, therapeutic or diagnostic purpose to be transferred, the inner sheath (4) being slidably received inside the outer sheath (3) and being flexible so as to be able to be extended, from the distal end of the outer sheath (3), into the uterine horn of the female mammal until the distal end of the inner sheath (4) is located in the transfer region; and
- a plunger (5; 5') intended, after placing inside the inner sheath (4) the material or substance to be transferred, to be inserted into the inner sheath (4) so as to be able to slide therein and to move, by a relative translation movement between the plunger (5; 5') and the inner sheath (4), the material or substance to be transferred towards the distal end of the inner sheath (4), through which the material or substance to be transferred will be placed in the transfer region,
the device (1) being **characterised in that** a part, so-called distal part, of the plunger (5; 5') is flexible and able to be extended into the part, so-called distal part (33), of the inner sheath (4) which is intended to be extended into the uterine horn, and **in that** the device (1) comprises joint movement means for jointly moving the inner sheath (4) and the plunger (5; 5') to the transfer region, with said distal part (33) of the plunger (5; 5') located inside said distal part of the inner sheath (4), and retraction means for retracting the inner sheath (4) on the plunger (5; 5') at the transfer region, so as to force out the material or substance to be transferred through the distal end of the inner sheath (4), under the push of the plunger (5; 5').

2. The device (1) according to claim 1, **characterised in that** the body assembly (2) comprises a shaft (11; 11') extending in the direction away from the outer sheath (3) and on which is slidably mounted a shuttle assembly (12) which comprises a primary shuttle (14), slidably mounted on said shaft (11; 11') and to which the plunger (5; 5') is attached, and a secondary shuttle (20), slidably mounted on said primary shuttle (14) and to which the proximal end of the inner sheath (4) is attached, the primary shuttle (14) and the secondary shuttle (20) constituting said joint movement means and said retraction means.

3. The device (1) according to one of claims 1 or 2, **characterised in that** the inner sheath (4) comprises a proximal part (32), inside the outer sheath (3), and the flexible distal part (33), preferably with a length greater than 130 mm, more preferably a length of 255.5 mm, intended to be extended into the uterine horn, the proximal part (32) being less flexible than the distal part (33).

4. The device (1) according to one of claims 1 to 3, suitable for the transfer of a material or substance with a reproductive, therapeutic or diagnostic purpose contained inside the inner space, with a given cross-section, of a straw (36), **characterised in that** the inner channel of the inner sheath (4) is able to receive such a straw (36), the plunger (5; 5') is able to pass through the straw (36) to force out the material or substance to be transferred (42) and the inner sheath (4) comprises translation stopping means for stopping the translation of the straw (36) in the direction of the distal end, the translation stopping means for stopping the translation of the straw (36) being formed, for example, by an inner shoulder (34) in the inner sheath (4), if appropriate located at the junction between the proximal (32) and distal (33) parts of the inner sheath (4) and, preferably, centered on the axis of the inner sheath (4).

5. The device (1) according to claim 4, the translation stopping means being formed by an inner shoulder (34) and for use with a straw (36) the inner space of which has a cross-section equal to the passage cross-section of the inner channel at the inner shoulder (34), **characterised in that** there are centering means for centering the straw (36) on the axis of the inner sheath (4) when the straw (36) is abutted against the translation stopping means, said centering means being formed, for example, by a narrowing (32a) of the passage cross-section of the inner channel of the inner sheath (4) at the vicinity of the shoulder (34), on the proximal end side of the inner sheath (4), the narrowing (32a) being centered on the axis of the inner sheath (4).

6. The device (1) according to one of claims 1 to 5, **characterised in that** the body assembly (2) comprises a nose (6) in which are provided a through passage (9), from an end of which extends the outer sheath (3), on a first side of the nose (6), and a through hole (10) opening onto a second side (8) of the nose (6), opposite said first side, and from which extends the shaft (11; 11') of the body assembly (2), the axis of the through passage (9) and the axis of the hole (10) being parallel and coplanar.

7. The device (1) according to one of claims 1 to 6, **characterised in that** the secondary shuttle (20) comprises a through passage (22) for the plunger (5; 5'), and **in that** the inner sheath (4) is connected to the secondary shuttle (20) by a connector (25), such as of the Luer type.

8. The device (1) according to one of claims 1 to 7, **characterised in that** the distal ends of the outer sheath (3) and of the inner sheath (4) are free of sharp edges and ridges, said distal ends being preferably rounded so as to facilitate moving forward the outer (3) and inner (4) sheathes inside the mammal without injuring the mammal.

9. The device (1) according to one of claims 1 to 8, **characterised in that** the distal end of the inner sheath (4) is formed by a tip (26) having two lateral outlets (35) allowing to place in the transfer region the material or substance to be transferred, such as semen towards the spermatic crypts, said tip (26) having preferably a rounded shape at the end thereof.

10. The device (1) according to one of claims 1 to 8, **characterised in that** the distal end of the outer sheath (3) is formed by a tip (47) made of flexible material, with a warhead shape and having a pierced cross-shaped opening (48).

11. The device (1) according to one of claims 1 to 10, **characterised in that** the plunger (5) is formed by a combination of a rod (43), for example made of metal, and a wire (44), respectively on the proximal side and the distal side of the device (1), the wire (44) forming the distal part of the plunger (5) intended to be extended into the part of the inner sheath (4) intended to be extended into the uterine horn of the mammal.

12. The device (1) according to claim 4, **characterised in that** the plunger (5') is formed by a rigid stem (46), forming the proximal part of the plunger (5') with which it is attached, if appropriate, to the primary shuttle (14), by a rod (43) which is flexible and able to pass through the straw (36), the rod (43), which is for example made of metal, extending from a distal end of the stem (46), and by a wire (44) extending from a distal end of the rod (43), the wire (44) and the distal end region of the rod (43) forming the distal part of the plunger (5') intended to be extended into the part of the inner sheath (4) intended to be extended into the uterine horn of the mammal.

13. The device (1) according to one of claims 1 to 12, **characterised in that** the length of the plunger (5; 5') is substantially equal to that of the inner sheath (4).

14. The device (1) according to one of claims 2 to 13, **characterised in that** the secondary shuttle (20) is provided with a first magnet (49), the primary shuttle (14) is provided, in the distal end region thereof, with a second magnet (50), and the shaft (11') is provided with a third magnet (51) in the proximal end region thereof, the magnets (49, 50, 51) being configured so as to be able to be aligned with each other in a given position of the primary (14) and secondary (20) shuttles and attract each other so as to hold in position the primary (14) and secondary (20) shuttles with respect to the shaft (11').

15. The device (1) according to one of claims 2 to 14, **characterised in that** the shaft (11; 11') carries, at the distal end thereof, a stop (13; 13'), preferably a stop (13') received in a through hole (17) of the primary shuttle (14), by which the primary shuttle (14) is slidably mounted on the shaft (11'), the through hole (17) comprising, preferably at the distal end thereof, a shoulder (15') having a stopping surface for said stop (13').
